# EUROPEAN PATENT APPLICATION

(11) **EP 2 221 055 A1**
(43) Date of publication of application: **25.08.2010**
(21) Application number: 09382020.7
(22) Date of filing: 18.02.2009
(51) Int. Cl.: A61K 31/472, A61P 11/06, A61P 11/08

(54) **5-(2-{[6-(2,2-difluoro-2-phenylethoxy)hexyl]amino}-1-hydroxyethyl)-8-hydroxyquinolin-2(1H)-one for the treatment of lung function**

(71) Applicant: Almirall, S.A., 08022 Barcelona (ES)
(72) Inventor: Ruf, Thorsten, 08950, ESPLUGUES DE LLOBREGAT (ES); Massana Montejo, Eric, 08006, BARCELONA (ES)
(74) Representative: Elzaburu Marquez, Alberto

(57) **Abstract**

A compound which is a hydroxyquinolinone derivative of formula (I), 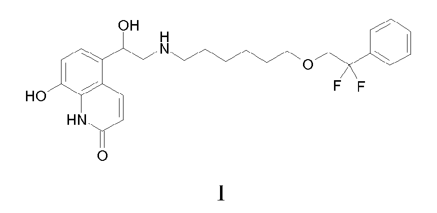 in the form of a racemate, a steroisomer or a mixture of stereoisomers, or a pharmaceutically acceptable salt or solvate thereof, for use in normalising the lung function of a human patient.

## Description

### Field of the Invention

The invention relates to methods for the normalisation of a patient's lung function *Background of the Invention*

5-(2-{[6-(2,2-difluoro-2-phenylethoxy)hexyl]amino}-1-hydroxyethyl)-8-hydroxyquinolin-2(1H)-one is a potent, long acting and selective β2 adrenergic receptor agonist. It is described in WO 2006/122788. It is a hydroxyquinolinone derivative of formula (I) below

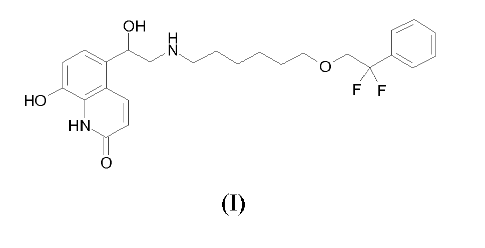

Salt forms of this compound are described in WO 2008/095720.

Currently available long acting β2 adrenergic receptor agonists include salmeterol (2-(hydroxymethyl)-4-{1-hydroxy-2-[6-(4-phenylbutoxy) hexylamino]ethyl}phenol) and formoterol (N-[2-hydroxy-5-[1-hydroxy-2-[1-(4-methoxyphenyl) propan-2-ylamino]ethyl] phenyl]formamide).

There are many ways in which a patient's lung function can be measured. These include measurement of a patient's peak flow, body plethysmography and spirometry. Spirometry involves measuring the amount and/or speed of air that can be inhaled or exhaled by a patient. A basic spirometry test generally involves a patient taking the deepest breath they can and then exhaling into a mouthpiece equipped with sensors that measure the volume and/or speed of air expelled. A useful measure of lung function that can be determined in this way is a patient's FEV1 value. FEV1 (Forced Expiratory Volume in 1 second) is the maximum amount of air that can be exhaled in one second by a patient. The reference FEV1 values which would be expected in a normal healthy person having a given weight and height are those published in the paper by Quanjer et al. in the European Respiratory Journal: Vol.6, Sup.16, pages 5-40, March 1993 and entitled "Lung Volumes and Forced Ventilatory Flows - Report Working Party - Standardization of Lung Function Test - European Community for Steel and Coal - Official Statement of the European Respiratory Society".

A decrease in lung function can be mediated by a number of different aetiologies. It is usually associated with respiratory disorders, such as asthma or Chronic Obstructive Pulmonary Disease (COPD), but it can also be produced by nervous or muscular disorders, such as muscular dystrophia, or even in situations like gastro-oesophageal reflux.

Patients suffering from a reduction in lung function usually experience discomfort and difficulty in breathing (dyspnoea), which can have a profound effect on the ability of a patient to tolerate exercise or to carry out his normal daily activities, particularly if it is caused by a chronic disease. In the long term a reduction in the ability to tolerate exercise can lead to obesity and cardiovascular disorders. It is also important to maintain an adequate lung function during the night. Low FEV1 values at night can cause episodes of nocturnal dyspnoea and sleep disorders. An acute decrease in lung function could be a life threatening situation in some patients and even produce death by asphyxia.

It is therefore desirable to treat patients so as to normalise their lung function, in particular increasing their FEV1 values towards the value that would be expected in a normal, healthy patient. Normalising the lung function of patients in this way can improve their quality of life both during the day and at night, ease discomfort and difficulty in breathing, and increase tolerance to exercise. A quick normalisation of lung function is also needed in case of severe acute dyspnoea and asphyxia.

It has been found that 5-(2-{[6-(2,2-difluoro-2-phenylethoxy)hexyl]amino}-1-hydroxyethyl)-8-hydroxyquinolin-2(1H)-one has a surprisingly high efficacy in increasing the lung function, and in particular the FEV1, of a patient experiencing a reduced lung function. 5-(2-{[6-(2,2-difluoro-2-phenylethoxy)hexyl]amino}-1-hydroxyethyl)-8-hydroxyquinolin-2(1H)-one can normalise the lung function and the FEV1 of a patient to substantially the value that would be expected in a normal, healthy person. The efficacy of 5-(2-{[6-(2,2-difluoro-2-phenylethoxy)hexyl]amino}-1-hydroxyethyl)-8-hydroxyquinolin-2(1H)-one in improving FEV1 is much larger than that observed with other known long acting beta agonists (LABAs), such as salmeterol.

### Summary of the Invention

The present invention therefore provides in a first embodiment a compound which is 5-(2-{[6-(2,2-difluoro-2-phenylethoxy)hexyl]amino}-1-hydroxyethyl)-8-hydroxyquinolin-2(1H)-one, in the form of a racemate, a stereoisomer or a mixture of stereoisomers, or a pharmaceutically acceptable salt or solvate thereof, for use by inhalation in normalising the lung function of a human patient.

According to particularly preferred embodiments of the present invention the compound of the invention is particularly useful for the treatment of patients with recalcitrant respiratory symptoms; in the maintenance therapy of patients not responding adequately to the treatment with bronchodilators, such as long-acting beta-adrenergic agonists; or in the treatment of patients with a severe decrease of lung function.

It is a further preferred embodiment of the invention that 5-(2-{[6-(2,2-difluoro-2-phenylethoxy)hexyl]amino}-1-hydroxyethyl)-8-hydroxyquinolin-2(1H)-one can be also used acutely as rescue medication, for example in respiratory emergencies.

### Detailed description of the invention

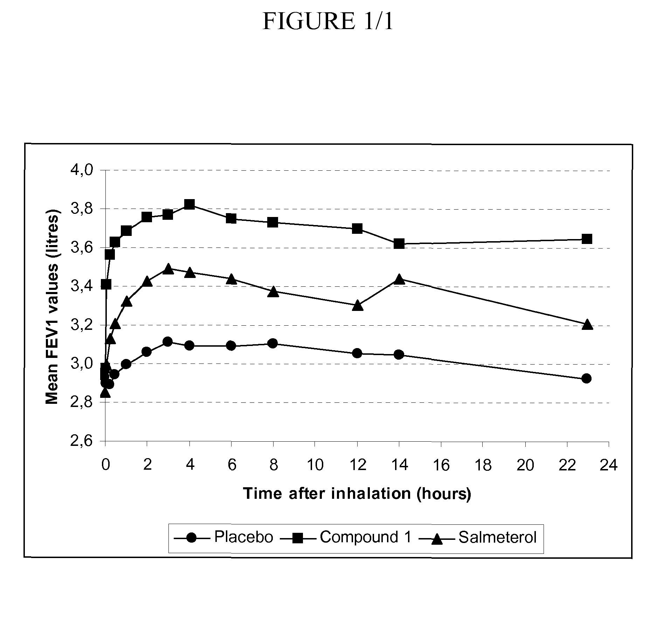

Figure 1 shows the mean FEV1 values (litres) over time (hours) for a single dose (25 µg) of 5-(2-{[6-(2,2-difluoro-2-phenylethoxy)hexyl]amino}-1-(R)-hydroxyethyl)-8-hydroxyquinolin-2(1H)-one referred to in the figure as "Compound 1". It also shows mean FEV1 values following two administrations, at time points 0 and 12 hours, of 50 µg of salmeterol and a single administration of placebo.

The term "therapeutically effective amount" refers to an amount sufficient to effect treatment when administered to a patient in need of treatment.

As used herein, "supramaximal dose" is a dose equal to or larger than the one which produces the maximal effect of a given compound.

The term "appropriate dose" refers to the dose which is deemed necessary for the treatment of a particular patient. It can be either a dose containing a "therapeutically effective amount" or a "supramaximal dose" depending on the disease, the patient, the initial level of lung function, the duration of the treatment or the frequency of administration.

The efficacy of 5-(2-{[6-(2,2-difluoro-2-phenylethoxy)hexyl]amino}-1-hydroxyethyl)-8-hydroxyquinolin-2(1H)-one is so high that it can be advantageously used in all kind of patients having a decreased lung function, independently of the aetiology. This includes patients with recalcitrant (i.e. difficult to treat) respiratory symptoms, such as persistent dyspnoea, cough or wheezing or patients with a particularly severe decrease in lung function, as defined below.

The compound of the invention is particularly useful in the continuous treatment of patients who have a chronic impairment of their lung function (also known as maintenance treatment), because it has a long duration of action and can be administered by inhalation only once a day. Moreover, due to its much higher efficacy compared to the drugs currently available for increasing lung function, such as beta-adrenergic agonists, anticholinergics or other bronchodilators, the compound of the invention can be used for treating patients who do not respond adequately to them. The example below shows that the compound of the invention produces a much larger effect on FEV1 than salmeterol.

It is considered that a patient does not respond adequately to treatment with a bronchodilator, or to other drugs used to improve lung function, when the patient continues to have symptoms or when the FEV1 values of the patient are lower than the "normalised" values described below, in spite of the treatment with the drug at the appropriate dose.

Typically, as used herein, a patient who does not respond adequately to treatment with bronchodilators is a patient who has an FEV1 value lower than the normalised values set out below, following twice daily treatment with 50 mcg salmeterol. A patient is also considered not to respond adequately to bronchodilators, and, more specifically, to beta-adrenergic agonists, when his FEV1 values do not increase by 200 ml or more after a single inhalation of 400 mcg of salbutamol.

Furthermore, thanks to its high efficacy and fast onset of action (clearly faster than the one of salmeterol) 5-(2-{[6-(2,2-dinuoro-2-phenylethoxy)hexyl]amino}-1-hydroxyethyl)-8-hydroxyquinolin-2(1H)-one has potentially life-saving benefits as rescue medication in respiratory emergency situations, for example in serious asthma attacks, exercise-induced bronchoconstriction or anaphylactic reactions, which may cause death by asphyxia. The use of the compound of the invention in these situations is particularly advantageous in comparison with other beta-adrenergic agonists commonly used as rescue medications, like salbutamol. The large and fast effect of the compound of the invention is sustained during a much longer period of time, which contributes to diminishing the recurrence of acute decreases of lung function and of further emergency situations.

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulphonic, benzoic, camphosulphonic, citric, ethanesulphonic, fumaric, gluconic, glutamic, hydrobromic, hydrochloric, lactic, maleic, malic, mandelic, methanesulphonic, mucic, naphthalene-1,5-disulphonic acid (napadisylate), nitric, pantothenic, phosphoric, succinic, sulphuric, tartaric, p-toluenesulphonic, xinafoic (1-hydroxy-2-naphthoic acid) and the like. Particularly preferred are salts derived from fumaric, hydrobromic, hydrochloric, acetic, sulphuric, methanesulphonic, naphthalene-1,5-disulphonic, xinafoic, and tartaric acids. Most preferred are salts derived from methanesulphonic and naphthalene-1,5-disulphonic acids.

Salts derived from naphthalene-1,5-disulphonic acid are typically mononapadisylate or heminapadisylate salts and pharmaceutically acceptable solvates thereof.

Typically, naphthalene-1,5-disulphonic acid salts of compounds of the invention have the formula (II): 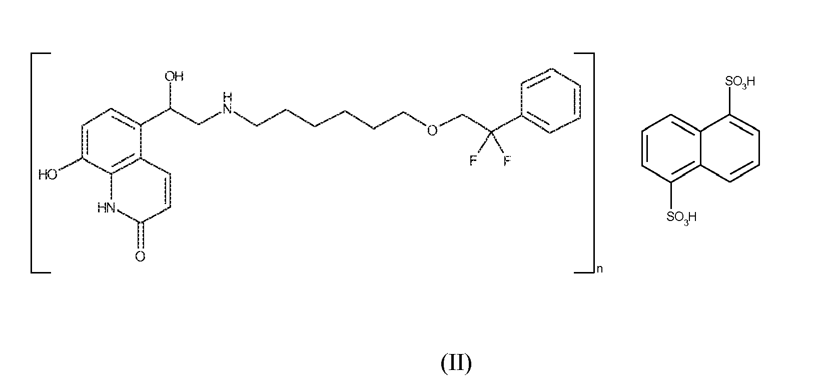 wherein n has a value of 1 or 2.

Salts derived from methanesulphonic acid are also known as mesylates

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

The term "solvate" refers to a complex or aggregate formed by one or more molecules of a solute, i.e. a compound of the invention or a pharmaceutically-acceptable salt thereof, and one or more molecules of a solvent. Such solvates are typically crystalline solids having a substantially fixed molar ratio of solute and solvent. Representative solvents include by way of example, water, methanol, ethanol, isopropanol, acetic acid, and the like. When the solvent is water, the solvate formed is a hydrate.

The compound of the invention contains a chiral center. Accordingly, it can be used in the form of a racemic mixture, an enantiomer, or a mixture enriched in one or more stereoisomer. The scope of the invention as described and claimed encompasses the racemic form of the compound of the invention as well as the individual enantiomers, and stereoisomer-enriched mixtures.

The most preferred enantiomer is 5-(2-{[6-(2,2-difluoro-2-phenylethoxy)hexyl]amino}-1-(R)-hydroxyethyl)-8-hydroxyquinolin-2(1H)-one.

It will be appreciated that the term "or a pharmaceutically acceptable salt or solvate thereof" is intended to include all permutations of salts and solvates, such as a solvate of a pharmaceutically-acceptable salt of a compound of the invention.

As used herein, reference to "normalising the lung function of a human patient" means in particular increasing the FEV1 of a patient having an FEV1 lower than the predicted normal values, as defined below, substantially to the predicted normal FEV1 for that patient.

The predicted normal FEV1 for a patient can be determined on the basis of the patient's age, height and sex. Thus, older patients typically have lower FEV1 values than younger patients of the same height and sex. Taller patients typically have higher FEV1 values than shorter patients of the same age and sex. Female patients typically have lower FEV1 values than male patients of the same age and height.

Determining a patient's predicted normal FEV1 is an entirely routine task for a thoracic physician. Typically, a patient's height, age and sex are used to determine the patient's normal FEV1 by an algorithm, or by consulting a standard table of predicted normal FEV1 values. Such tables are widely available and are based in the study by Quanjer et al., 1993, mentioned above. According to this study the predicted FEV1 of a normal human being can be calculated by means of the following formulae:
- Men: 4.3 H - 0.029 A - 2.49
- Women: 3.95 H - 0.025 A - 2.6
wherein H = height (metres) and A= age (years)

Typically, as used herein, the predicted normal FEV1 of a patient is determined by reference to these formulae.

A patient's FEV1 is typically determined experimentally by spirometry. Determining FEV1 by spirometry is a standard technique well known in the art of thoracic medicine. In assessing lung function following administration of a medicament, a patient's FEV1 is generally determined at regular time intervals after administration. Particularly useful measurements that can be made when determining the efficacy of a medicament are the baseline FEV1, the peak FEV1 and the trough FEV1.

As used herein the baseline FEV1 is the FEV1 of a patient before administration of a medicament.

As used herein the peak FEV1 is the maximum FEV1 of a patient during the first three, four or five hours after administration of a medicament. For example, to determine the maximum FEV1 in the first three hours after administration of a medicament, regular measurements of a patient's FEV1 are made in the first three hours, for example once every 30 minutes.

As used herein the trough FEV1 is the FEV1 of a patient measured between 22 and 24 hours after administration of a medicament, e.g. a compound of the invention. 23 hours is an appropriate time point to measure trough FEV1 of a compound suitable for once-daily administration, though equally acceptable alternative time points in this case are 22 or 24 hours after administration. The average of the FEV1 values measured at 23 and 24 hours after administration is also a usual reference for trough FEV1.

The patient whose lung function is normalised by the compound of the invention typically has a baseline FEV1 (i.e. before administration of the compound) which is less than 90% of the predicted normal FEV1 for that patient. In particular embodiments, the baseline FEV1 is less than 85%, or 80% or 75% of the predicted normal FEV1 for that patient. In yet other embodiments, the baseline FEV1 is less than 70%, or 65%, or 60% of the predicted normal FEV1 for that patient.

In the case of the clinical trial described below the baseline FEV1 of the patients was 61 % to 85% of the predicted values, which corresponds to patients having mild to moderate persistent asthma, according to the guidelines published by GINA (Global Initiative for Asthma) in Bateman et al., Eur. Resp. J., 31(1):143.178, 2008.
In view of the very high efficacy of the compound observed in this trial with mild /moderate patients it is clear that a group of patients that would especially benefit from the invention are those having a particularly severe decrease in lung function. Following the GINA guidelines on FEV1 values, which can be applied to decreases of lung function of any aetiology, this would be the case of patients having a baseline FEV1 equal or lower than 60 % of the predicted normal FEV1 for that patient.

From a clinical perspective, the lung function of a population of human patients is typically "normalised" if (depending on the severity of the disease, the baseline FEV1 values and the duration of the treatment) the average FEV1 of the patients is increased to no less than a certain percentage lower than the average predicted normal FEV1 for that group of patients. Normalisation of the lung function and of FEV1 is also achieved when the average FEV1 value of the group of patients is increased by a certain volume (in ml or in percentage) over the average baseline FEV1 of the patients or over the average FEV1 value obtained when the patients are administered placebo. Accordingly, the specific FEV1 values described in the following paragraphs for individual patients should be interpreted from this clinical perspective.

Thus, the lung function and the FEV1 of a human patient is typically "normalised" if (depending on the severity of the disease, the baseline FEV1 values and the duration of the treatment) the average peak FEV1 of the patient is increased to no less than 30% lower than the predicted normal FEV1 for that patient, preferably to no less than 25%, or 20% or 15% lower than the predicted normal FEV1 for that patient, more preferably to no less than 10% or 5% lower than the average predicted normal FEV1 for that patient.

In the context of a once-daily administration, the compound of the invention would typically be administered in the morning. Thus, the normalised peak FEV1 values would be achieved within a few hours (i.e. within two to five hours), at the time of the day when the patient needs to have a maximal lung function, adequate for carrying out his normal daily activities.

Further, the lung function and the FEV1 of a human patient is typically "normalised" if (depending on the severity of the disease and on the baseline FEV1 values), the trough FEV1 of the patient is increased to no less than 40% lower than the predicted normal FEV1 for that patient, preferably to no less than 35%, or 30% or 25 % lower than the predicted normal FEV1 for that patient, more preferably no less than 15%, or 10% lower than the predicted normal FEV1 for that patient.

In the context of a once-daily administration these normalised trough FEV1 values would be sufficient to allow the patient have a restful sleep without episodes of nocturnal dyspnoea and without requiring any additional administration of a bronchodilator.

As mentioned above, the compound of the present invention normalises lung function by increasing a patient's peak and trough FEV1 values above the patient's baseline FEV1 before administration of a compound of the invention. The compound of the present invention also normalises lung function by increasing a patient's peak and trough FEV1 values above the peak and trough FEV1 values obtained when the patient is administered placebo.

Typically, the compound of the invention (administered at the appropriate doses as a single dose or in a continued once- or twice-daily inhalation) increases the peak FEV1 of the patient (depending on the severity of the disease, the baseline FEV1 values and the duration of the treatment), by more than 400 ml over the baseline FEV1 of the patient. Preferably, administration of a compound of the invention increases the peak FEV1 by more than 450, 500 or 550 ml over the baseline FEV1. More preferably, by more than 600, 650, or 700 ml. Most preferably, the compound of the invention increases the peak FEV1 by more than 750 or 800 ml over the baseline FEV1, particularly when given as a single supramaximal dose.

Typically, the compound of the invention (administered at the appropriate doses as a single dose or in a continued once- or twice-daily inhalation) increases the peak FEV1 of a patient (depending on the severity of the disease, the baseline FEV1 values and the duration of the treatment) by more than 9%, over the baseline FEV1 of the patient. Preferably, administration of a compound of the invention increases the peak FEV1 by more than 12%, 15% or 18% over the baseline FEV1. More preferably, by more than 21% or 24 %. Most preferably, the compound of the invention increases peak FEV1 by more than 27% over the baseline FEV1, particularly when given as a single supramaximal dose.

Typically, the compound of the invention (administered at the appropriate doses as a single dose or in a continued once- or twice-daily inhalation) increases the trough FEV1 of a patient (depending on the severity of the disease, the baseline FEV1 values and the duration of the treatment) by more than 150 ml over the baseline FEV1 of the patient. Preferably, administration of a compound of the invention increases the trough FEV1 by more than 200, 250 or 300 ml over the baseline FEV1. More preferably, by more than 350, 400 or 450 ml. Most preferably, the compound of the invention increases trough FEV1 by more than 500, 550 or 600 ml over the baseline FEV1, particularly when given as a single supramaximal dose.

Typically, the compound of the invention (administered at the appropriate doses as a single dose or in a continued once- or twice-daily inhalation) increases the trough FEV1 of a patient (depending on the severity of the disease, the baseline FEV1 values and the duration of the treatment) by more than 5% over the baseline FEV1 of the patient. Preferably, administration of a compound of the invention increases the trough FEV1 by more than 7%, 9% or 11% over the baseline FEV1. More preferably, by more than 13%, 15% or 17%. Most preferably, the compound of the invention increases trough FEV1 by more than 19%, 21% or 23 % over the baseline FEV1, particularly when given as a single supramaximal dose.

Typically, the compound of the invention (administered at the appropriate doses as a single dose or in a continued once- or twice-daily inhalation) increases the peak FEV1 of a patient (depending on the severity of the disease, the baseline FEV1 values and the duration of the treatment) by more than 250 ml over the peak FEV1 measured when the patient is administered placebo. Preferably, administration of a compound of the invention increases the peak FEV1 by more than 300, 350 or 400 ml over the peak FEV1 of the placebo. More preferably, by more than 450 or 500 ml. Most preferably, the compound of the invention increases peak FEV1 by more than 550 or 600 ml over the peak FEV1 of the placebo, particularly when given as a single supramaximal dose.

Typically, the compound of the invention (administered at the appropriate doses as a single dose or in a continued once- or twice-daily inhalation) increases the peak FEV1 of a patient (depending on the severity of the disease, the baseline FEV1 values and the duration of the treatment) by more than 7% over the peak FEV1 measured when the patient is administered placebo. Preferably, administration of a compound of the invention increases the peak FEV1 by more than 9%, 11 % or 13% over the peak FEV1 of the placebo. More preferably, by more than 15% or 17%. Most preferably, the compound of the invention increases peak FEV1 by more than 19% over the peak FEV1 of the placebo, particularly when given as a single supramaximal dose.

Typically, the compound of the invention (administered at the appropriate doses as a single dose or in a continued once- or twice-daily inhalation) increases the trough FEV1 of a patient (depending on the severity of the disease, the baseline FEV1 values and the duration of the treatment) by more than 100 ml over the trough FEV1 measured when the patient is administered placebo. Preferably, administration of a compound of the invention increases the trough FEV1 by more than 150, 200, 250, 300 or 350 ml over the trough FEV1 of the placebo. More preferably, by more than 400, 450, 500 or 550 ml. Most preferably, the compound of the invention increases trough FEV1 by more than 580, 620 or 660 ml over the trough FEV1 of the placebo, particularly when given as a single supramaximal dose.

Typically, the compound of the invention (administered at the appropriate doses as a single dose or in a continued once- or twice-daily inhalation) increases the trough FEV1 of a patient (depending on the severity of the disease, the baseline FEV1 values and the duration of the treatment) by more than 3% over the trough FEV1 measured when the patient is administered placebo. Preferably, administration of a compound of the invention increases the trough FEV1 by more than 5%, 7% or 9% over the trough FEV1 of the placebo. More preferably, by more than 11%, 13% or 15%. Most preferably, the compound of the invention increases trough FEV1 by more than 17%, 19% or 21% over the trough FEV1 of the placebo, particularly when given as a single supramaximal dose.

Typically, the compound of the invention is administered by inhalation. The compound of the invention is preferably administered by inhalation from an inhaler or a nebuliser.

Typically, the compound of the invention is in a pharmaceutical composition comprising any suitable excipients or pharmaceutically acceptable carriers and in the form of a dry powder or a solution suitable for inhalation.

Typically, the compound of the invention is administered at an amount per inhalation equivalent to a metered nominal dose of 25 µg or less administered with a dry powder inhaler. The term "metered nominal dose" refers to the quantity of drug substance contained in the metering chamber of the delivery device and is normally expressed as quantity per inhalation. Upon actuation, the drug substance leaves the device and becomes available to the patient as a so-called "emitted dose", which is normally smaller than the metered nominal dose, due to the mechanics of the device.

Typically, the compound of the invention is administered as a single-dose treatment or in continued treatments with one or more doses per day, preferably from 1 to 4 doses per day, more preferably from 1 to 2 doses per day, even more preferably as 1 dose per day.

Typically, the hydroxyquinolinone derivative of formula (I) is chosen from (R,S) 5-(2-{[6-(2,2-difluoro-2-phenylethoxy)hexyl]amino}-1-hydroxyethyl)-8-hydroxyquinolin-2(1H)-one and 5-(2-{[6-(2,2-difluoro-2-phenylethoxy)hexyl]amino}-1(R)-hydroxyethyl)-8-hydroxyquinolin-2(1H)-one.

Preferably, the hydroxyquinolinone derivative of formula (I) is present as the R-enantiomer.

Alternatively, the hydroxyquinolinone derivative of formula (I) is present as the S-enantiomer.

Typically, the compound of the invention is a pharmaceutically acceptable salt of a hydroxyquinolinone derivative of formula (I) as defined herein. Preferably, the compound of the invention is a mesylate, mononapadisylate or heminapadisylate salt of a hydroxyquinolinone derivative of formula (I) as defined herein.

Preferably, the hydroxyquinolinone derivative of formula (I) is selected from one of 5-(2-{[6-(2,2-difluoro-2-phenylethoxy)hexyl]amino}-1(R)-hydroxy-ethyl)-8-hydroxyquinolin-2(1H)-one heminapadisylate, or a pharmaceutically acceptable solvate thereof, and 5-(2-{[6-(2,2-difluoro-2-phenylethoxy)hexyl]amino}-1(R)-hydroxy-ethyl)-8-hydroxyquinolin-2(1H)-one mesylate, or a pharmaceutically acceptable solvate thereof.

Typically, the compound is co-administered with a therapeutically effective amount of another therapeutic agent.

The other therapeutic agent is typically a corticosteroid, an anticholinergic agent, and/or a PDE4 inhibitor.

Examples of suitable PDE4 inhibitors are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid (MK-0873), CDC-801, UK-500001, BLX-914, 2-carbomethoxy-4-cyano-4-(3-cyclopropylmethoxy-4-difluroromethoxyphenyl)cyclohexanl-one, *cis* [4-cyano-4-(3-cyclopropylmethoxy-4-difluoromethoxyphenyl)cyclohexan-1-ol, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO03/097613, WO2004/058729, WO 2005/049581, WO 2005/123693 and WO 2005/123692.

Examples of suitable corticosteroids and glucocorticoids are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acctoxy]-4-pregnene-3,20-dione, Desisobutyrylciclesonide, hydrocortisone acetate, hydrocortisone sodium succinate, NS-126, prednisolone sodium phosphate and hydrocortisone probutate, Prednisolone sodium metasulfobenzoate and clobetasol propionate

Examples of suitable M3 antagonists (anticholinergics) are tiotropium salts, oxitropium salts, flutropium salts, ipratropium salts, glycopyrronium salts, trospium salts, zamifenacin, revatropate, espatropate, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3 (R)-ylmethyl] carbamoyl] ethyl] carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazohdin-1-yl]-1-methyl-1-(2-phenylethyl)pyrrolidmium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Particularly preferred additional therapeutic agents are selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO03/097613, WO2004/058729, WO 2005/049581, WO 2005/123693 and WO 2005/123692.

Thus, one aspect of the invention provides a compound of the invention as defined herein, and a corticosteroid for simultaneous, concurrent separate or sequential use in normalising the lung function, and in particular the FEV1, of a human patient as defined herein.

Also provided is a compound of the invention for use in normalising the lung function, and in particular the FEV1, of a human patient as defined herein, in which the compound of the invention is co-administered with a corticosteroid.

Also provided is a corticosteroid for use in normalising the lung function, and in particular the FEV1, of a human patient as defined herein, by co-administration with a compound of the invention.

Particularly preferred corticosteroids are those selected from the group consisting of mometasone furoate, ciclesonide, budesonide, fluticasone furoate and fluticasone propionate.

Another aspect of the invention provides a compound of the invention as defined herein, and an anticholinergic agent and, optionally, a corticosteroid for simultaneous, concurrent separate or sequential use in normalising the lung function, and in particular the FEV1, of a human patient as defined herein.

Also provided is a compound of the invention as defined herein for use in normalising the lung function, and in particular the FEV1, of a human patient as defined herein, in which the compound of the invention is co-administered with an anticholinergic agent and, optionally, a corticosteroid.

Also provided is an anticholinergic agent for use in normalising the lung function, and in particular the FEV1, of a human patient as defined herein by co-administration with a compound of the invention and, optionally, a corticosteroid.

Particularly preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]octane salts. Optional corticosteroids are preferably selected from the group consisting of mometasone furoate, ciclesonide, budesonide, fluticasone furoate and fluticasone propionate.

A still further aspect of the invention provides a compound of the invention as defined herein, and a PDE4 inhibitor and, optionally, a corticosteroid, and/or an anticholinergic agent, for simultaneous, concurrent separate or sequential use in normalising the lung function, and in particular the FEV1, of a human patient as defined herein.

Also provided is a compound of the invention for use in normalising the lung function, and in particular the FEV1, of a human patient as defined herein, in which the compound of the invention is co-administered with a PDE4 inhibitor and, optionally, a corticosteroid, and/or an anticholinergic agent.

Also provided is a PDE4 inhibitor for use in normalising the lung function, and in particular the FEV1, of a human patient as defined herein by co-administration with a compound of the invention and, optionally, a corticosteroid, and/or an anticholinergic agent.

Particularly preferred PDE4 inhibitors are those selected from the group consisting of rolipram, roflumilast, cilomilast and the compounds claimed in the PCT patent applications number WO03/097613, W02004/058729, WO 2005/049581, WO 2005/123693 and WO 2005/123692. Optional corticosteroids are preferably selected from the group consisting of mometasone furoate, ciclesonide, budesonide, fluticasone furoate and fluticasone propionate. Optional anticholinergic agents are preferably selected from the group consisting oftiotropium salts, glycopirronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

A particularly preferred embodiment of the present invention provides a compound of the invention as defined herein, and a therapeutically effective amount of a 3-[2-Hydroxy-2,2-bis(2-thienyl)acetoxy]-1-(3-phenoxypropyl)-1-azoniabicyclo[2.2.2]octane salt and, optionally, a corticosteroid and/or a PDE4 inhibitor for simultaneous, concurrent separate or sequential use in normalising the lung function, and in particular the FEV1, of a human patient as defined herein.

Also provided is a compound of the invention as defined herein for use in normalising the lung function, and in particular the FEV1, of a human patient as defined herein in which the compound of the invention is co-administered with a therapeutically effective amount of a 3-[2-Hydroxy-2,2-bis(2-thienyl)acetoxy]-1-(3-phenoxypropyl)-1-azoniabicyclo[2.2.2]octane salt and, optionally, a corticosteroid and/or a PDE4 inhibitor.

Also provided is a therapeutically effective amount of a 3-[2-Hydroxy-2,2-bis(2-thienyl)acetoxy]-1-(3-phenoxypropyl)-1-azoniabicyclo[2.2.2]octane salt for use in normalising the lung function, and in particular the FEV 1, of a human patient as defined herein by co-administration with a compound of the invention and, optionally, a corticosteroid and/or a PDE4 inhibitor.

Another particularly preferred embodiment of the present invention provides a compound of the invention as defined herein, and a therapeutically effective amount of mometasone furoate and, optionally, an anticholinergic and/or a PDE4 inhibitor for simultaneous, concurrent separate or sequential use in normalising the lung function, and in particular the FEV1, of a human patient as defined herein.

Also provided is a compound of the invention as defined herein for use in normalising the lung function, and in particular the FEV1, of a human patient as defined herein in which the compound of the invention is co-administered with a therapeutically effective amount of mometasone furoate and, optionally, an anticholinergic and/or a PDE4 inhibitor.

Also provided is a therapeutically effective amount of mometasone furoate for use in normalising the lung function, and in particular the FEV 1, of a human patient as defined herein by co-administration with a compound of the invention and, optionally, an anticholinergic and/or a PDE4 inhibitor.

Yet another embodiment of the invention provides a compound of the invention as defined herein, a corticosteroid, an anticholinergic agent and a PDE4 inhibitor for simultaneous, concurrent separate or sequential use in normalising the lung function, and in particular the FEV1, of a human patient as defined herein.

Typically, the patient is suffering from a respiratory disease, such as asthma or COPD, and/or a disease which decreases lung function.

Typically, the pharmaceutical compositions comprising a compound of the present invention and a pharmaceutically acceptable carrier are suitable for administration by inhalation and may further comprise a therapeutically effective amount of one or more other therapeutic agents, as defined herein. However, any other form of topical, parenteral or oral application is possible. The application of inhaled dosage forms embodies the preferred application form, especially in the therapy of diseases or disorders of the lung.

The pharmaceutical compositions may be prepared by any of the methods well known in the art of pharmacy. All methods include the step of bringing the active ingredient(s) into association with the carrier. In general the pharmaceutical compositions are prepared by uniformly and intimately bringing into association the active ingredient(s) with liquid carriers or finely divided solid carriers or both and then, if necessary, shaping the product into the desired form.

The carrier for a pharmaceutical composition in the form of a dry powder is typically chosen from starch or a pharmaceutically acceptable sugar, such as lactose or glucose. Lactose is preferred.

Additional suitable carriers can be found in Remington: The Science and Practice of Pharmacy, 20th Edition, Lippincott Williams & Wilkins, Philadelphia, Pa., 2000.

The pharmaceutical compositions for inhalation are delivered with the help of inhalers, such as dry powder inhalers, aerosols or nebulisers. The inhaler is typically configured to deliver, upon actuation, a therapeutically effective amount of one or more other therapeutic agents, as defined herein.

Packaging of the compound of the invention in the inhaler may be suitable for unit dose or multi-dose delivery. In the case of multi-dose delivery, the compound of the invention can be pre-metered or metered in use.

The inhaler is typically a single dose inhaler, a multiple unit dose inhaler, or a multi dose device inhaler.

Dry powder compositions for topical delivery to the lung by inhalation may, for example, be presented in capsules and cartridges of for example gelatine or blisters of for example laminated aluminium foil, for use in an inhaler or insufflator.

Dry powder inhalers are classified into three groups: (a) single dose, (b) multiple unit dose and (c) multi dose devices.

For inhalers of the first type (a), single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e.g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers (b) together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose devices (c) do not contain pre-measured quantities of the medicament containing powder. They consist of a relatively large container and a dose measuring principle that has to be operated by the patient. The container bears multiple doses that are isolated individually from the bulk of powder by volumetric displacement. Various dose measuring principles exist, including rotatable membranes (e.g. EP0069715) or disks (e.g. GB 2041763; EP 0424790; DE 4239402 and EP 0674533), rotatable cylinders (e.g. EP 0166294; GB 2165159 and WO 92/09322) and rotatable frustums (e.g. WO 92/00771), all having cavities which have to be filled with powder from the container. Other multi dose devices have measuring plungers with a local or circumferential recess to displace a certain volume of powder from the container to a delivery chamber or an air conduit (e.g. EP 0505321, WO 92/04068 and WO 92/04928), or measuring slides such as the Novolizer SD2FL (ex. Sofotec), also known as Genuair®, which is described in the following patent applications: WO 97/000703, WO 03/000325 and WO 03/061742.

Reproducible dose measuring is one of the major concerns for multi dose devices.
The powder formulation has to exhibit good and stable flow properties, because filling of the dose measuring cups or cavities is mostly under the influence of the force of gravity. For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (e.g. WO 97/000703). For such semi-permanent multi dose inhalers with disposable drug containers, the requirements to prevent drug accumulation are even more strict.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes, the particles of the active ingredient as produced may be size reduced by conventional means e.g. by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Apart from applications through dry powder inhalers the compositions of the invention can also be administered in nebulisers, metered dose inhalers and aerosols which operate via propellant gases or by means of so-called atomisers, via which solutions of pharmacologically-active substances can be sprayed under high pressure so that a mist of inhalable particles results. Such atomisers are described, for example, in W0 91/14468 and WO 97/12687.

These liquid formulations generally contain a suitable carrier which may be either a propellant for MDI administration or water for administration through a nebuliser. The formulation may comprise additional components such as preservatives (for example, benzalkonium chloride, potassium sorbate, benzyl alcohol); pH stabilizers (for example, acidic agents, alkaline agents, buffer systems); isotonic stabilizers (for example, sodium chloride); surfactant and wetting agents (for example, polysorbates, sorbitan esters); and/or absorption enhancers (for example, chitosan, hyaluronic acid, surfactants). The formulation may also contain additives to improve the solubility of other active compounds when mixed with the salt of the invention. The solubility enhancers may comprise components such as cyclodextrins, liposomes or co-solvents such as ethanol, glycerol and propylene glycol.

Additional suitable carriers for formulations of the active salts of the present invention can be found in Remington: The Science and Practice of Pharmacy, 20th Edition, Lippincott Williams & Wilkins, Philadelphia, Pa., 2000.

Pressurized aerosol compositions will generally be filled into canisters fitted with a valve, especially a metering valve. Canisters may optionally be coated with a plastics material e.g. a fluorocarbon polymer as described in W096/32150. Canisters will be fitted into an actuator adapted for buccal delivery.

The present invention also provides a method of normalising the lung function, and in particular the FEV1, of a human patient, which method comprises administering to said patient a therapeutically effective amount of a compound which is 5-(2-{[6-(2,2-difluoro-2-phenylethoxy)hexyl]amino}-1-hydroxyethyl)-8-hydroxyquinolin-2(1H)-one, i.e. a hydroxyquinolinone derivative of formula (I), 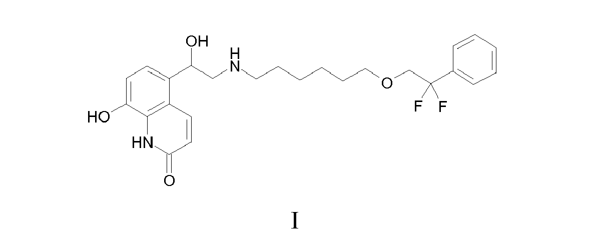 in the form of a racemate, a steroisomer or a mixture of stereoisomers, or a pharmaceutically acceptable salt or solvate thereof.

The present invention also provides use of a compound which is 5-(2-{[6-(2,2-difluoro-2-phenylethoxy)hexyl]amino}-1-hydroxyethyl)-8-hydroxyquinolin-2(1H)-one, i.e. a hydroxyquinolinone derivative of formula (I), 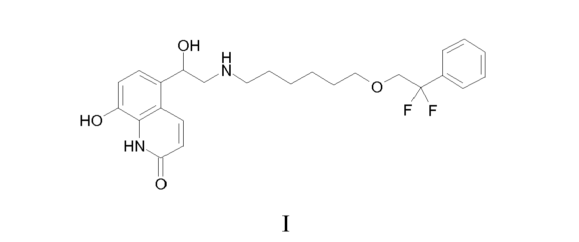 in the form of a racemate, a stereoisomer or a mixture of stereoisomers, or a pharmaceutically acceptable salt or solvate thereof, in the manufacture of a medicament for use in normalising the lung function, and in particular the FEV1, of a human patient.

### Example 1

Clinical Phase II study: A randomised double-blind, double-dummy, placebo and active comparator-controlled, cross-over trial assesses the activity, safety, tolerability and pharmacokinetics of single doses of 5-(2-{[6-(2,2-difluoro-2-phenylethoxy)hexyl]amino}-1-(R)-hydroxyethyl)-8-hydroxyquinolin-2(1H)-one heminapadisylate by inhalation in asthma patients.

Methods: Men with a diagnosis of mild to moderate persistent asthma, as defined by the 2006 GINA guideline, for at least 6 months prior to screening and with a FEV1 61-85% of the predicted normal values (according to Quanjer et al. 1993) were randomised to treatment sequences comprising a single-dose administration of 5-(2-{[6-(2,2-difluoro-2-phenylethoxy)hexyl]amino}-1-(R)-hydroxyethyl)-8-hydroxyquinolin-2(1H)-one heminapadisylate (at a metered nominal dose of 25 micrograms in the Cyclohaler® device), two administrations (at time points 0 and 12 hours) of Salmeterol (at a metered nominal dose of 50 micrograms in the Cyclohaler® device) and placebo administered all of them by means of a dry powder inhaler. Lung function measurements included (by spirometry) FEV1, PEF, FVC, FEF25-75, and (by body plethysmography) sGaw and Raw.

Results: 25 males (aged 18 to 70) took part in the study. 5-(2-{[6-(2,2-difluoro-2-phenylethoxy)hexyl]amino}-1-(R)-hydroxyethyl)-8-hydroxyquinolin-2(1H)-one (25 micrograms) clearly increased the average peak and trough FEV1 compared with placebo and with salmeterol. Strikingly, the increase in average trough FEV1 is statistically significant compared to salmeterol, even though this compound was administered in two doses. The non-corrected descriptive data obtained are shown in the tables below.

**Peak FEV1**

| Treatment | Baseline (pre-dose) FEV1 (L) | Peak FEV1 (L) | Increase over baseline (mL) | Increase over placebo (mL) |
|---|---|---|---|---|
| 5-(2-{[6-(2,2-difluoro-2-phenylethoxy)hexyl]amino}-1-(R)-hydroxyethyl)-8-hydroxyquinolin-2(1H)-one (25 mcg) | 2.976 | 3.819 | 843 | 618 |
| Salmeterol (50 mcg) | 2.854 | 3.521 | 667 | 442 |
| Placebo | 2.928 | 3.153 | 225 | ----- |

**Trough FEV1**

| Treatment | Baseline (pre-dose) FEV1 (L) | Trough FEV1 (L) | Increase over baseline (mL) | Increase over placebo (mL) |
|---|---|---|---|---|
| 5-(2-{[6-(2,2-difluoro-2-phenylethoxy)hexyl]amino}-1-(R)-hydroxyethyl)-8-hydroxyquinolin-2(1H)-one (25 mcg) | 2.976 | 3.642 | 666 | 718 |
| Salmeterol (50 mcg) | 2.854 | 3.209 | 355 | 285 |
| Placebo | 2.928 | 2.924 | - 004 | ----- |

This remarkable normalisation of lung function, as shown by the FEV1 values above, is observed over the whole period of 24 hours, as shown in Figure 1, and resulted in clinically relevant effects. Similar trends are seen with PEF, FVC, FEF25-75, sGaw and Raw.

Conclusion: A single dose of 5-(2-{[6-(2,2-difluoro-2-phenylethoxy)hexyl]amino}-1-(R)-hydroxyethyl)-8-hydroxyquinolin-2(1H)-one (25 micrograms) produces a surprisingly large, long-lasting bronchodilation, with a fast onset, which results in a remarkable normalisation of lung function. This is demonstrated by the high FEV1 values obtained, which are significantly higher than those produced by salmeterol.

## Claims

1. A compound which is 5-(2-{[6-(2,2-difluoro-2-phenylethoxy)hexyl]amino}-1-hydroxyethyl)-8-hydroxyquinolin-2(1H)-one in the form of a racemate, a stereoisomer or a mixture of stereoisomers, or a pharmaceutically acceptable salt or solvate thereof, for use by inhalation in normalising the lung function of a human patient.

2. A compound which is 5-(2-{[6-(2,2-difluoro-2-phenylethoxy)hexyl]amino}-1-hydroxyethyl)-8-hydroxyquinolin-2(1H)-one in the form of a racemate, a stereoisomer or a mixture of stereoisomers, or a pharmaceutically acceptable salt or solvate thereof, for use by inhalation as maintenance therapy in chronic respiratory patients not responding adequately to the treatment with bronchodilators.

3. A compound which is 5-(2-{[6-(2,2-difluoro-2-phenylethoxy)hexyl]amino}-1-hydroxyethyl)-8-hydroxyquinolin-2(1H)-one in the form of a racemate, a stereoisomer or a mixture of stereoisomers, or a pharmaceutically acceptable salt or solvate thereof, for use by inhalation as rescue medication in respiratory emergency situations.
